(19) Europäisches Patentamt

European Patent Office

Office européen des brevets

(11) **EP 1 721 634 A1**

(12) **EUROPEAN PATENT APPLICATION**
published in accordance with Art. 158(3) EPC

(43) Date of publication:
**15.11.2006 Bulletin 2006/46**

(51) Int Cl.:
***A61N 5/06*** (2006.01)

(21) Application number: **05710131.3**

(22) Date of filing: **10.02.2005**

(86) International application number:
**PCT/JP2005/002079**

(87) International publication number:
**WO 2005/077458 (25.08.2005 Gazette 2005/34)**

(84) Designated Contracting States:
**AT BE BG CH CY CZ DE DK EE ES FI FR GB GR HU IE IS IT LI LT LU MC NL PL PT RO SE SI SK TR**

(30) Priority: **13.02.2004 JP 2004037392**

(71) Applicant: **Fancl Corporation Kanagawa 231-0806 (JP)**

(72) Inventors:
 • **TAMURA, Shigeaki**
 c/o Fancl R & D Center
 Yokohama-shi,Kanagawa 2440806 (JP)

 • **ENOMOTO, Akiko**
 c/o Fancl R & D Center
 Yokohama-shi,Kanagawa 2440806 (JP)
 • **UDA, Masaki**
 c/o Fancl R & D Center
 Yokohama-shi,Kanagawa 2440806 (JP)

(74) Representative: **Vossius & Partner
 Siebertstrasse 4
 81675 München (DE)**

(54) **METHOD OF CHEMILUMINESCENCE-UTILIZING MAKEUP AND BEAUTIFICATION, LUMINANT FOR SKIN IRRADIATION BEAUTIFICATION AND MAKEUP/ BEAUTIFICATION EQUIPMENT**

(57) Presentation of the methods for makeup and beauty treatment by chemical luminescence, luminous bodies for beauty treatment by exposure to skin, and tools for makeup and beauty treatment

FIG.9

EP 1 721 634 A1

**Description**

Technical Field

This invention relates to methods for makeup and beauty treatment, luminous bodies for beauty treatment by exposing to the skin, and tools for makeup and beauty treatment by the use of chemical luminescence- the emission of low capacity.

Background Technology

There has been no technical idea to utilize the light for the purpose of skin care and skin activation. In particular, the chemical luminescence has not been applied to makeup and beauty treatment.

In the field of beauty treatment, the light has been regarded as one of the causes to damage the skin and therefore should be blocked against the skin. With such concept, a lot of cosmetic products have been proposed to block the light or to protect the skin against it. As an exceptional case, the light has been used to get a tan positively at the tanning parlor. Further, there has been an example to apply the laser light to the affected part of the body for the purpose of medical treatment.

The examples of the proposal to use the powerful lights like the laser light are as follows.

Patent document 1 (Patent publication No. 2004-733) Laser depilation equipment. Patent document 2 (Patent publication No. 2003-12487) Laser treatment for the purposes like depilation by exposing to the laser light the skin pre-coated with the beauty lotion.

Patent document 3 (Patent publication No. H11-342213) The tape to promote laser light absorption and also the beauty treatment using the said tape to allow build-up of the beautiful skin with high efficiency by increasing the laser light absorption at the positions to be cared on the skin, in particular on the face skin, and by assuring a certain level of absorption effect for promoting the metabolism.

Most of the application of the light is for the medical purpose, except for enhancing the tanning. The strong light like the sunlight is not positively used for application to beauty care or to makeup, as it would cause skin inflammation, pigmentation and skin aging after tanning.

Patent document: Patent publication No. 2004-733

Patent document: Patent publication No. 2003-12487

Patent document 3: Patent publication No. H11-342213)

Disclosure of Invention

Problem to Be Solved by the Invention

The inventors of this invention found out the effect of the light on the skin by chemical luminescence in the course of the research work to develop a new method of beauty treatment and makeup and accomplished this invention. This invention offers new methods for beauty treatment and makeup as well as new tools for beauty treatment and makeup, reagents for makeup and beauty treatment, by making use of chemical luminescence.

Means for Solving Problem

This invention relates to offering the effect of beauty treatment and makeup by putting close to or in contact with the skin the chemical luminescent bodies that emit the light for several minutes to several hours without producing high temperature heat evolution by mixing two liquid components.

(1) A method for beauty treatment or makeup by exposing a skin to the light obtained by chemical luminescence.

(2) The method for beauty treatment or makeup described in (1), characterized in that the light is selected from the wavelength range of 300 to 1200nm.

(3) The method for beauty treatment or makeup described in (1) or (2), characterized in that the skin is a part of the face or the body.

(4) The method for beauty treatment or makeup described in any one of (1) to (3), characterized in that mixing two liquid components generates the chemical luminescence.

(5) The method for beauty treatment or makeup described in any one of (1) to (4), characterized in that the light is irradiated onto an applied area after an external use skin medicine is applied or while a pack agent is being applied.

(6) The method for beauty treatment or makeup described in (5), characterized in that the external use skin medicine or the pack agent comprises a composition for beauty treatment or for makeup.

(7) A luminous body for irradiating a skin for beauty treatment or makeup, characterized in that a chemical luminescent reagent is contained.

(8) The luminous body for irradiating the skin for beauty treatment or makeup described in (7), characterized in that the chemical luminescent reagent constituted by two liquid components that emits light upon mixing, is stored separately in one container and mixed to expose the skin upon use.

(9) The luminous body for irradiating the skin for beauty treatment or makeup described in (7) or (8), characterized in that the light for irradiating the skin is selected from the wavelength range of 300 to 1200nm.

(10) A tool for beauty treatment or makeup, characterized in that an external use skin medicine or oral medicine is used in combination with a luminous body for irradiating the skin for beauty treatment or makeup described in (7), (8) or (9).

(11) A method for whitening makeup or whitening beauty treatment by exposing a skin to the light of chemical luminescence.

(12) The method for whitening makeup or whitening beauty treatment described in (12), characterized in that the growth of the melanin producing cells and / or the synthesis of melanin is inhibited by exposing the skin to the light of chemical luminescence.

(13) The method for whitening makeup or whitening beauty treatment described in (11) or (12),
characterized in that a luminous color of the chemical luminescence comprises one or a plurality of a combination selected from blue fluorescent light, yellow fluorescent light, green fluorescent light, orange fluorescent light and red fluorescent light.

(14) A whitening luminous body containing a luminescent reagent that emits the light of chemical luminescence to a skin.

(15) The luminous body described in (14), characterized in that the luminescent reagent inhibits the growth of melanin producing cells and /or the synthesis of melanin by emitting the light of chemical luminescence to the skin.

(16) The luminous body described in (14) or (15), characterized in that the luminescent reagent emits fluorescent light comprising one or a plurality of a combination selected from blue fluorescent light, yellow fluorescent light, green fluorescent light, orange fluorescent light and red fluorescent light.

(17) The method for whitening makeup or whitening beauty treatment described in any one of (11) to (13), characterized in that the blue fluorescent light is irradiated onto the skin as chemical luminescence while olive leaf extract is being applied on the skin, being applied as a pack agent or after taking it orally.

(18) A tool for whitening makeup or whitening beauty treatment, characterized by comprising a combination of a whitening luminous body containing a chemical luminescent reagent of blue fluorescent light and an external use skin medicine, a pack agent and / or an oral medicine containing olive leaf extract.

(19) A beauty treatment method or a makeup method, characterized in that the growth of fibroblast and / or the synthesis of collagen is promoted by exposing a skin to the light of chemical luminescence comprising one or a plurality of orange fluorescent light, red fluorescent light, green fluorescent light, and yellow fluorescent light.

(20) The method for beauty treatment or makeup according to claim 19, characterized in that the growth of fibroblast and / or the synthesis of collagen is promoted by exposing the skin to the light of chemical luminescence comprising one or a plurality of orange fluorescent light, red fluorescent light, green fluorescent light, and yellow fluorescent light while ascorbic acid, its salt and / or a derivative of ascorbic acid are being applied on the skin, being applied as a pack agent or after taking it orally.

(21) A luminous body, characterized in that a luminescent reagent that emits fluorescent light comprising one or a plurality of orange fluorescent light, red fluorescent, green fluorescent, and yellow fluorescent as the light of chemical luminescence to promote the growth of fibroblast and / or the synthesis of collagen by exposing a skin is contained.

(22) A tool for beauty treatment or makeup, characterized in that the tool comprises a combination of the luminous body described in (21) and with an external use skin medicine, a pack agent and / or an oral medicine containing ascorbic acid, or its salt and / or a derivative of ascorbic acid.

(23) A light anti-aging method by exposing a skin to blue fluorescent light.

(24) The light anti-aging method described in (23), characterized in that generating pyridine dimer is inhibited by exposing the skin to the blue fluorescent light of chemical luminescence.

(25) The light anti-aging method described in (23) or (24), characterized in that the skin is exposed to the blue fluorescent light while photolyase is being applied on the skin, being applied as a pack agent or after taking it orally.

(26) A luminous body for light anti-aging, characterized by containing a chemical fluorescent reagent to irradiate a skin with blue fluorescent light.

(27) A light anti-aging tool for makeup or beauty treatment, characterized by comprising a combination of a chemical luminescent reagent that radiates blue fluorescent light to a skin and an external use skin medicine, a pack agent and / or an oral medicine containing photolyase.

(28) A method to increase blood flow by exposing a skin to fluorescent light of chemical luminescence and / or near-infrared rays of chemical luminescence.

(29) The method to increase the blood flow described in (28), characterized in that the fluorescent light of the chemical luminescence is orange fluorescent light.

(30) A luminous body to increase blood flow, which contains a chemical luminescent reagent that emits fluorescent light and / or near-infrared rays.

(31) The luminous body to increase the blood flow described in (30), characterized in that the fluorescent light is orange fluorescent light.

(32) A tool for makeup or beauty treatment, characterized by comprising a combination of a luminous body to increase the blood flow described in (30) or (31) with an external use skin medicine.

(33) The tool for makeup or beauty treatment described in (10), (18), (22), (27), or (32), characterized in that the external use skin medicine is skincare cosmetics, facial pack, poultice, patches, or transdermal absorbents.

(34) A skin masking material, characterized in that any one of the luminous body or the tool for beauty treatment or makeup described in (7), (8), (9), (10), (14), (15), (16), (18), (21), (22), (26), (27), (30), (31), or (32) is used for face masking or body masking.

Effect of the Invention

(1) As this invention uses the light of chemical luminescence, which is low in energy and does not emit heat, it is safe and does not damage the skin and human body.

(2) The use of chemical luminescent reagents consisting of two liquid components, allowing emitting the light upon mixing, presents the features like excellent portability, no restriction in use as to place, occasion and convenience.

(3) Chemical luminescence allows the control of wavelength as the wavelength can be chosen from the broad wavelength range from ultraviolet to infrared. Further, luminescence of high intensity is also usable.

(4) In the design of the chemical luminescentbody, there is a choice in size to cover from a point to a large area to match the place and space where it is to be used.

(5) This invention can be used extensively as luminescence time can be controlled from several minutes to over 10 hours.

(6) There is a cell activation action · effect.

(7) This invention can be used in combination with cosmetics·quasi drug, transdermal absorbent, internal medicine (oral drug). The combination may offer synergy effect.

(8) This invention offers the synergy effect when used in combination with beauty treatment components or chemical components.

(9) This invention presents the whitening effect. In particular, the whitening function was confirmed due to the property to inhibit the growth of melanin producing cells or the synthesis of melanin. In particular, blue fluorescent light, green fluorescent light, yellow fluorescent light, orange fluorescent light and red fluorescent light give the positive effect for whitening.

(10) Even when the skin is exposed to the ultraviolet light like sunshine, skin blackening or generation of stains or freckles can be inhibited by exposing the skin to fluorescent light afterward as it inhibits the synthesis of melanin.

(11) By combining blue fluorescent light irradiation with application of olive leaf extract, it is possible to increase synergistically the effect to inhibit the growth of human epidermal melanocyte.

(12) Exposing the skin to the light of chemical luminescence is useful for anti-aging. In particular, irradiation with orange fluorescent light, red fluorescent light or green fluorescent light promotes the growth of fibroblast and the synthesis of collagen, and the said growth and synthesis can be still more promoted with synergistic effect by using ascorbic acid or its derivative at the same time.

(13) Exposing the skin to the light of chemical luminescence presents the light anti-aging effect. In particular, irradiation with blue fluorescence inhibits the damage of DNA in the skin cell by ultraviolet light, and serves to light anti-aging. The effect can be still more enhanced by using photolyase at the same time.

(14) Exposing the skin to the light of chemical luminescence to the skin presents the effect to improve the blood circulation. In particular, irradiation with near-infrared rays or orange fluorescent light serves to increase the blood flow.

(15) The chemical luminescent body of this invention is compact and light, allows the flexibility in design of size, thickness, softness etc., and has little restriction in its use. It can stick to the skin by using adhesives.

Brief Description of Drawings

[Fig. 1 (a)] Graph of spectral radiance for red fluorescent light

[Fig. 1 (b)] Graph of spectral radiance for green fluorescent light

[Fig. 1 (c)] Graph of spectral radiance for blue fluorescent light

[Fig. 1 (d)] Graph of spectral radiance for yellow fluorescent light

[Fig. 2] Graph of spectral radiance for high intensity fluorescent light

[Fig. 3] Graph of illumination change for normal fluorescent light

[Fig. 4] Graph of illumination change for high intensity fluorescent light

[Fig. 5] Graph of observation for blood tube width change

[Fig. 6] Graph showing the growth rate of cells

[Fig. 7] Graph showing the effect of fluorescence irradiation on the growth of B16 cells

[Fig. 8] Graph showing the effect of fluorescence irradiation on the synthesis rate of melanin

[Fig. 9] Graph showing how melanin synthesis is inhibited in the human three dimensional skin model by irradiation of high intensity blue fluorescent light

[Fig. 10] Graph showing the growth of human epidermal melanocyte by irradiation with high intensity blue fluorescent light

[Fig. 11] Graph showing the growth rate of fibroblast

[Fig. 12] Graph showing the synthesis rate of collagen

[Fig. 13] Graph showing the growth rate of fibroblast (culture medium containing 1% blood serum) in irradiation with individual fluorescent light colors

[Fig. 14] Graph showing the growth rate of fibroblast (culture medium containing 5% blood serum) in irradiation with individual fluorescent light colors

[Fig. 15] Graph showing the growth rate of fibroblast in irradiation with high intensity fluorescent light

[Fig. 16] Graph showing the effect of promoting photolyase activation by irradiation with high intensity blue fluorescent light

[Fig. 17] Photo spectrograph of near-infrared rays of chemical luminescent body

[Fig. 18] Output graph of near-infrared rays of chemical luminescent body

[Fig. 19] Image showing the blood flow

[Fig. 20] Graph showing the blood flow by irradiation with near-infrared fluorescence

Best Modes for Carrying Out the Invention

This invention has the effect to act on whitening, anti-aging, anti- light-aging, blood flow improvement, skin temperature increase at the exposure spot and its neighborhood, and growth of cells by irradiating with the light of chemical luminescence. The effect changes with the wavelength and the radiance of the light to be irradiated.

Such effect can be applied to beauty treatment, makeup or medical use independently. Further, the absorption can be promoted by combination with transdermal absorbents or orally taken absorbents.

As the light of chemical luminescence does not generate heat and has the property to adapt to shape, size and roughness of the skin, application time and durability, it is highly safe and convenient.

The effect of chemical luminescence is controllable by adjusting wavelength, intensity, and the durable time by means of the composition of luminescent reagent. The variety of luminescence extends to various fluorescent light colors as well as to infrared rays.

In terms of the specific application to the beauty treatment field, this invention is useful as remedies to stains, freckles, dullness, darkness, tension, wrinkles, and bag of the skin, which grows with the aging.

The aging phenomenon is caused for reasons like fading of reddish skin by feeble blood circulation, diffuse deposition of melanin, shade due to the rough skin surface created by deteriorated elasticity of the skin, fading of luster by random reflection on the skin surface, and skin yellowing due to aging. These phenomena show up typically as "dullness of the skin", have no distinct boundaries to each other and cannot be covered by makeup.

The aforementioned irradiation with the light of chemical luminescence can handle the problems like dullness and aging as it improves transparency and brightness through better blood circulation, promotes activation of metabolism, and activates cellular growth.

The exposure of the skin to chemical luminescence can be applied to cosmetics, quasi medicine, makeup reagent, beauty reagent, makeuptool, beautytool, medical instrument, skin beauty, curing of sports damage, and curing of muscle fatigue.

Further, as other applications, the high intensity luminous body is useful for delicate adjustment of daily rhythm (circadian rhythm) and also for adjustment of autonomous nerves. Its effect to promote cellular proliferation offers suitable application to the tool for curing the wound. This can be used as luminous wound band and makeup kit for curing pimples. The effect to raise the skin temperature and to improve the blood flow serves to promote metabolism, and thereby waste products can be removed and growth of hair is promoted. By combining this function with the transdermal absorbent, the effect of the transdermal absorbent can be enhanced. For example, the combination with the face mask for skin care or with the poultice is effective.

The chemical fluorescent reagent of this invention can be spread to the thin and flexible cover band, and the said band can be used with its luminous surface in direct contact with the skin. It is desirable to cover the luminous surface with the photo-transmitting film. The size of the cover band bearing the chemical fluorescent reagent can be decided in accordance with the part to be applied. Further, non-woven or sponge can be used as the carrier for the luminous body. It facilitates to soften the whole area or the surface to get in contact with the skin, thereby to ease the close contact with the skin. An example of the softening material is the soft and transparent film of artificial resin like silicone resin.

It was observed that the exposure of the skin to chemical luminescence gives whitening effect. Unlike ultraviolet light, chemical luminescence does not promote the synthesis of melanin by the emission of fluorescent light, and has the effect to inhibit the proliferation of the melanin producing cells and the synthesis of melanin in the cell.

This effect has been confirmed by chemical luminescence including blue fluorescent light, yellow fluorescent light, green fluorescent light, orange fluorescent light and red fluorescent light.

The effect to inhibit the synthesis of melanin was observed by blue fluorescent light and yellow fluorescent light, in particular the synthesis of melanin was suppressed by 20% only by blue fluorescent light.

In the case of red fluorescent light, high intensity green fluorescent light and high intensity orange fluorescent light, it was observed that the proliferation of melanocyte was suppressed, suggesting that the total amount of melanin is reduced by the reduction of the number of cells.

Irradiation with fluorescence has the effect of whitening as it suppresses selectively the proliferation of the activated melanocyte, inhibiting the proliferation of melanin producing cells as well as the synthesis of melanin in the cell. This means that irradiation with chemical luminescence is useful for whitening makeup and whitening beauty treatment.

It was confirmed effective to use chemical luminescence in combination with transdermal absorbent by testing with olive leaf extract, and by using blue as fluorescent light.

The proliferation of human epidermal melanocyte was observed to drop by irradiation with high intensity blue fluorescent light when olive leave extract is kept absorbed through the skin or taking it orally. The combination of spreading or oral taking of olive leaf extract and the irradiation with high intensity blue fluorescent light inhibits the proliferation of human epidermal melanocyte, and thus it is considered to have the whitening effect. Olive leaf extract can be used as a transdermal absorbent in the form of makeup component in liquid state or pack component.

By exposing the skin to irradiation with chemical fluorescence, the synthesis of melanin by UV light irradiation can be inhibited, and accordingly it shows whitening effect and beauty treatment effect by inhibiting blackening. UVB irradiation promotes the synthesis of melanin. It was confirmed that irradiation with high intensity blue fluorescent light suppresses the melanin synthesis by UVB irradiation. It implies that, even when the skin is exposed to UV light like sunshine, emergence of blackening, stains and freckles can be inhibited by suppressing the melanin synthesis by exposing the skin later on to fluorescent of chemical luminescence such as high intensity blue fluorescent light

It was observed that irradiation with chemical luminescence inhibited aging of the skin. Aging of the skin takes place when the regeneration power of fibroblast declines or the synthesis power of collagen drops.

It was confirmed that the exposure of the skin to irradiation with chemical luminescence had the function to promote proliferation of fibroblast and synthesis of collagen. Chemical luminescence includes orange fluorescent light, red fluorescent light, green fluorescent light, and yellow fluorescent light.

For example, irradiation with orange fluorescent light, red fluorescent light, green fluorescent light, yellow fluorescent light, high intensity orange fluorescent light, or high intensity green fluorescent light promotes proliferation of fibroblast and synthesis of collagen in the fibroblast cells.

It was confirmed that the combined application of chemical luminescence with transdermal absorbent or oral administration absorbent had effect to promote proliferation of fibroblast and synthesis of collagen. For the combination component, a derivative of ascorbic acid was employed.

Irradiation with chemical luminescence to the skin is useful for anti-aging as it increases the amount of collagen in the skin, effecting to enhance the tension and to improve wrinkles, elasticity and softness. Still better, synergistic effect can be obtained by using together the reagent containing a derivative of ascorbic acid and the like. The said reagent can be in the form of liquid makeup reagent, pack reagent or oral administration reagent.

It was recognized that irradiation with chemical luminescence inhibits light-aging of the skin.

When the skin is exposed to UVB, DNA is damaged and pyrimidine dimer is produced. It is said that the repetition of the said process induces the progress of light-aging. The prokaryote having the ability of photosynthesis bears "photolyase" which can repair dimerization of such DNA base. It is confirmed that activation of photolyase can be realized by irradiation with chemical luminescence.

For example, irradiation with high intensity blue fluorescent light demonstrated the effect to reduce the content of pyrimidine dimer.

Further, by using high intensity blue fluorescent light together with photolyase capable of enhancing the reduction of the pyrimidine dimer content, the pyrimidine dimer content can be reduced synergistically and the effect to suppress light-aging is enhanced even more.

It was recognized that irradiation with a variety of fluorescent light shows the effect to promote the proliferation of fibroblast.

Irradiation with chemical luminescence under the condition of poor nutrition demonstrated to promote the proliferation of fibroblast, and thus the function of chemical luminescence was confirmed. For example, cell activation effect was recognized by normal type red fluorescent light, green fluorescent light, and yellow fluorescent light as well as by high intensity orange fluorescent light, and high intensity green fluorescent light. The most effective fluorescent color light is green.

The increase of blood flow rate could be recognized by irradiation with chemical luminescence.

Irradiation with chemical luminescence to the skin has the effect to increase blood circulation.

Direct measurement was made to confirm the improvement of the blood flow and the rise of the skin temperature. It allows the promotion of metabolism, transdermal absorption, and the effect of oral administration to act on the skin subjected to exposure of irradiation.

<Chemical fluorescent body>

Chemical luminescence is generated by using two component system to emit luminescence chemically. It is the common practice to generate chemical luminescence by the chemical reaction of two components, namely "oxalate" component (salt or ester of oxalic acid) and "activator" component in the state of chemical solution.

The said two components are separated physically by a variety of means before activation. In many cases, a glass container of fragile sealing that contain one component is held in a soft outer container containing another component. The outer container is sealed to keep inside both the second component and glass container of fragile filling. When the glass container is broken by the force of strong contact with the inner vial, for example by the force generated by bending, the first component escapes and gets mixed with the second component, and resultantly emits luminescence. Since the objective of the device to generate chemical luminescence is to gain enough output for practical use, the outer container consists of the transparent or semitransparent material such as polyethylene, polypropylene or silicone resin, which normally allows radiation from chemical luminescence system to pass through the container wall. Luminescence of different colors can be generated in accordance with the composition for chemical luminescence. This device can be designed in such a way that the light passes through the specifically selected section.

For the purpose of the covering element to constitute the container, soft film can be employed as it adapts well to the site of exposure.

In relation to chemical luminescence, there are already many proposals. For example, there are references including the following Japanese Patents: Patent publication No. 2003-238823 (Composition for chemical luminescence) Patent publication No. 2002-138278 (Chemical luminescence system), Patent publication No. H11-045602 (Chemical fluorescentbody), and Indication of existence of Patent 2003-532253 (Luminous elements by chemical luminescence).

The composition of the chemical luminescent body consists of liquid A containing fluorescent reagent and liquid B containing oxidant. Liquid A consists of ester, fluorescent substance, dim ethyl phthalate, and so on, while liquid B consists of hydrogen peroxide, catalyst, butyl phthalate and so on. Liquid A and liquid B are held in one container in the separated state and mixing is effected to initiate luminous reaction when required.

As two components, hydrogen peroxide is used as one component and ester of oxalic acid is used as another for the purpose of chemical luminescentbody. For chemical luminescence catalyst, the catalyst for two-component chemical luminescence system is employed such as lithium carbonate, specifically lithium salicylate for example. The catalyst for two-component chemical luminescence system reduces the activation energy for the reaction and decreases the temperature dependence of luminescence process.

One of the components can be immersed to non-woven or sponge in the actual application as it improves the softness and ensures the better contact with rough surface of the skin.

More specifically, the components that can be used for chemical luminescent reaction are disclosed in Patent publication No. 2003-238823 and described below. The solution for chemical luminescence reaction should contain the component like oxalate or activator. For this invention, oxalate (salt or ester of oxalic acid) used normally for chemical luminescence reaction can be employed without any particular limitation. Suitable examples include bis(2,6-dichloro-4-nitrophenyl) oxalate, bis(2,4,6-trichlorophenyl) oxalate, bis(3-trifluoromethyl-4-nitorphenyl) oxalate, bis(2-methyl-4,6-dinitrophenyl) oxalate, bis(1,2-dimethyl-4,6-dinitrophenyl) oxalate, bis(2,4-dichlorophenyl) oxalate, bis(2,5-dinitrophenyl) oxalate, bis(2-formyl-4-nitrophenyl) oxalate, bis(pentachlorophenyl) oxalate, bis(1,2-dihydro-2-oxo-1-pyridyl)glyoxal, bis-N-phthalmydil oxalate, bis(2,4,5-trichloro-6-carbobenthoxyphenyl) oxalate, bis(2,4,5-trichloro-6-carbobutoxyphenyl) oxalate, bis(2,4,6-trichlorophenyl) oxalate, and bis(2,4,5-trichloro-6-pentoxyphenyl) oxalate.

As activator, peroxide component is normally employed. Suitable examples include solution of hydrogen peroxide compound, hydrogen peroxide compound, or solution of hydrogen peroxide compound diluted with dilute solution. The said hydrogen peroxide compound includes hydrogen peroxide and compound to generate hydrogen peroxide.

Preferably, hydrogen peroxide is used as peroxide and it can be used as solution. Alternatively, anhydride of peroxide compound, such as sodium per borate and sodium peroxide can also be used. Further, it is allowed to use any compound to generate hydrogen peroxide. As solvent to form activator solution, there is no specific restriction, but preferably triethyl citrate or dim ethyl phthalate is used.

As the first polymer resin particle and the second polymer resin particle, a variety of polymers can be used. Examples include, but not limited to, polyethylene, polypropylene, polyvinyl chloride, polymethyl methacrylate, polyvinyl benzoate, polyvinyl acetate, cellulose polyvinyl pyrolydon, polyacryl amide, epoxides, silicones, polyvinyl butylal, polyurethane, nylons, polyacetal, polycarbonate, polyesters and polyethers.

Further, bridged polymers can also be used, such as polystyrene-polydivinylbenzen, polyacryamide-polymethylenebisacrylamide, and polybutadiene copolymer. Above all, polyvinyl chloride resin is preferable.

As fluorescent reagent, the components described below can be employed as illustrated in Patent publication 2002-138278. Examples include the conjugated polycyclic aromatic compounds containing at least three fused rings, such as anthracene, substituted anthracene, benzoanthracene, phenanthrene, substituted phenanthrene, naphtacene, substituted naphthacene, pentacene, substituted pentacene, perylene, substituted perylene, violanthrone, and substituted violanthrone.

Examples of substituent groups include phenyl group, lower alkyl group (C1-C16), chloro group, bromo group, cyano group, and alcoxy group (C1-C16).

Suitable examples of fluorescent substance include 1-methoxy-9,10 bis(phenylethynyl) anthracene, 1-methoxy-9,10-bis(phenylethynyl)anthracene, perylene, 1,5-dichloro-9,10bis(phenylethynyl)anthracene, 1,8-dichloro-9,10 bis(phenylethynyl) anthracene, rubrene, monochloro and dichloro substituted 9,10- bis(phenylethynyl) anthracene, 5,12-bis (phenylethynyl)tetracene, 9,10 diphenyl anthracene, 16,17-dihexyloxyviolanthrone, 2-methyl-9,10- bis(phenylethynyl) anthracene, 9,10-(4-methoxyphenyl)-2-chloroanthracene, 9,10-bis-(4-ethoxyphenyl)-2-chloroanthracene, 16.17-didecichloxyviolanthrone, "LUMOGEN RED" (perylenedicarboxyimide fluorescent agent to emit red fluorescent light), "LUMOGEN YELLOW" (perylenedicarboxyimide fluorescent agent to emit yellow fluorescent light), "LUMOGEN ORANGE" (perylenedicarboxyimide fluorescent agent to emit orange fluorescent light), 5,12-bis-(phenylethynyl)naphtacene, 5,6,11,12-tetraphenyl naphtacene or mixtures thereof.

By using the composition described above, luminescence can be obtained with wavelengths ranging from 300 to 1200nm. Further, it becomes possible to control the luminescence reaction speed as well by adding catalyst. In an example of this invention, an experiment is shown using fluorescence of two types consisting of high and low intensity and near-infrared rays. For the purpose of convenience, fluorescence of high radiance is called high intensity type.

Since the laser wavelength range utilized currently for medical treatment is from 560 to 1200nm, chemical luminescence covers this wavelength range quite well. The followings are the examples already put into practical use; IPL(Intense Pulsed Light):560-1200nm, LLLT(low reactive level laser therapy):830nm, laser wavelengths for the therapy of sports damage: 630nm, 780nm, 800nm, 830nm, 904nm.

<Chemical luminescent reagent>

In the examples of this invention, fluorescence of two types differing in radiance intensity is used, and for the purpose of convenience, a type with high radiance is called "high intensity type", and another with low intensity "normal type".

In the examples of this invention, normal type fluorescence consisting of five varieties including CYALUME Light shape blue, Light shape green, Light shape yellow, Light shape orange, and Light shape red produced by OmniGlow Japan Co., Ltd. are used as well as high intensity type consisting of five varieties including "high intensity type blue fluorescent light", "high intensity type green fluorescent light", "high intensity type yellow fluorescent light", "high intensity type orange fluorescent light", and "high intensity type red fluorescent light".

The said five varieties of normal type showed the peak of spectral radiance as described below; red fluorescent light over $800 \times 10^{-5}$W/(sr·m$^2$·nm) at wavelength 605nm, yellow fluorescent light over $260 \times 10^{-5}$W/(sr·m$^2$·nm) at 550nm, green fluorescent light over $450 \times 10^{-5}$ W/(sr·m$^2$·nm) at 510nm, and blue fluorescent light over $260 \times 10^{-5}$W/(sr·m$^2$·nm) at 450nm.

The said five varieties of high intensity type showed the peak of spectral radiance as described below; high intensity red fluorescent light over 0.006W/(sr·m$^2$·nm) at wavelength 660nm, high intensity orange fluorescent light over 0.009W/ (sr·m$^2$·mn) at 625nm, high intensity yellow fluorescent light over 0.011 W/(sr·m$^2$·nm) at 555nm, high intensity green fluorescent light over 0.015W/(sr·m$^2$·nm) at 520nm, and high intensity blue fluorescent light over 0.007W/(sr·m$^2$·nm) at 450nm.

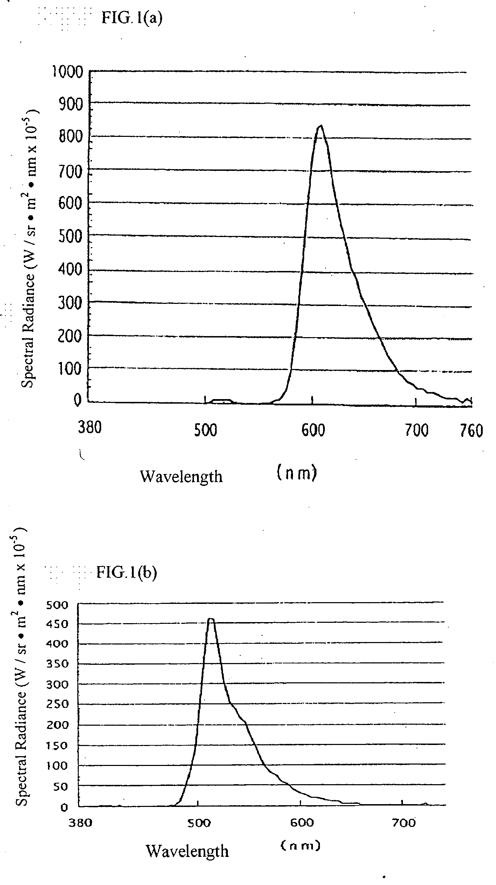

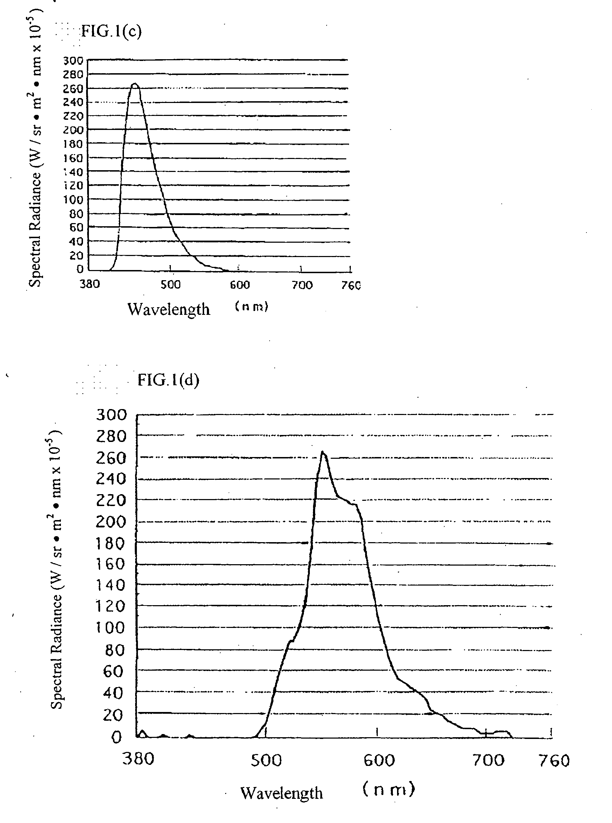

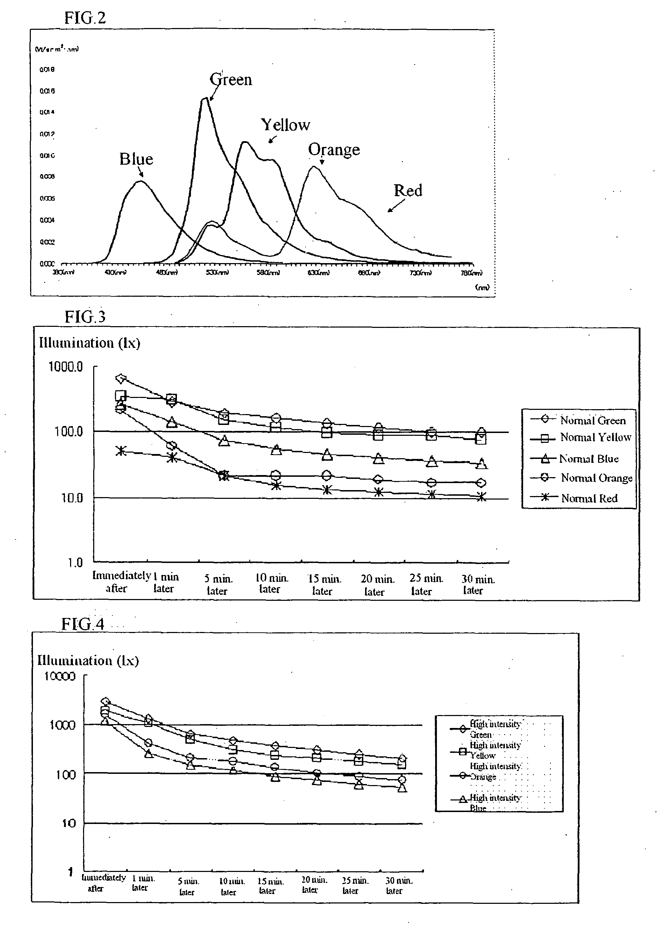

The examples of fluorescent light measurement are shown in the form of graph in Fig. 1(a) to Fig. 1(d) for the normal type fluorescent light and in Fig. 2 for the high intensity type.

<Properties of fluorescent light irradiation>

The change pattern of fluorescence illumination with time is shown in Table 1, Table 2 Fig. 3 and Fig.4. Table 1 and Fig. 3 show the values for green fluorescent light, blue fluorescent light, orange fluorescent light, and red fluorescent light of normal type. Table 2 and Fig. 4 show the values for green fluorescent light, orange fluorescent light, blue fluorescent light, and red fluorescent light of high intensity type. Fluorescence with properties described here is used in examples 2 to 7.
[Table 1]

| | immediately after | 1Min.later | 5Min.later | 10Min.later | 15Min.later | 20Min.later | 25Min.later | 30Min.later |
|---|---|---|---|---|---|---|---|---|
| Illumination change with time for normal fluorescence, elapsed time (min.), illumination unit lx | | | | | | | | |
| Normal Green | 651.0 | 295.0 | 195.0 | 162.0 | 134.0 | 115.0 | 100.0 | 100.0 |
| Normal Yellow | 351.3 | 310.0 | 152.0 | 117.7 | 98.3 | 92.3 | 91.7 | 77.3 |
| Normal Blue | 268.0 | 141.0 | 74.4 | 54.2 | 45.2 | 40.5 | 36.8 | 33.7 |
| Normal Orange | 221.0 | 60.1 | 22.1 | 22.1 | 22.0 | 19.0 | 17.0 | 17.0 |
| Normal Red | 50.9 | 40.1 | 22.1 | 15.4 | 13.1 | 12.5 | 11.5 | 10.7 |

[Table 2]

| Illumination change with time for high radiance fluorescence, elapsed time (min.), illumination unit 1x | | | | | | | | |
|---|---|---|---|---|---|---|---|---|
| | immediately after | 1Min.later | 5Min.later | 10Min. later | 15Min. later | 20Min. later | 25Min. later | 30Min. later |
| High intensity Green | 2940 | 1319 | 671 | 482 | 378 | 311 | 253 | 219 |
| High intensity Yellow | 1900 | 1063.3 | 505.3 | 313.3 | 248.0 | 214.3 | 182.5 | 160.0 |
| High intensity Orange | 1633 | 403 | 217 | 180 | 136 | 106 | 88 | 75 |
| High intensity Blue | 1166.7 | 260.3 | 157.0 | 117.3 | 89.3 | 76.3 | 62.4 | 55.2 |

EXAMPLE 1

<Improvement of blood flow>

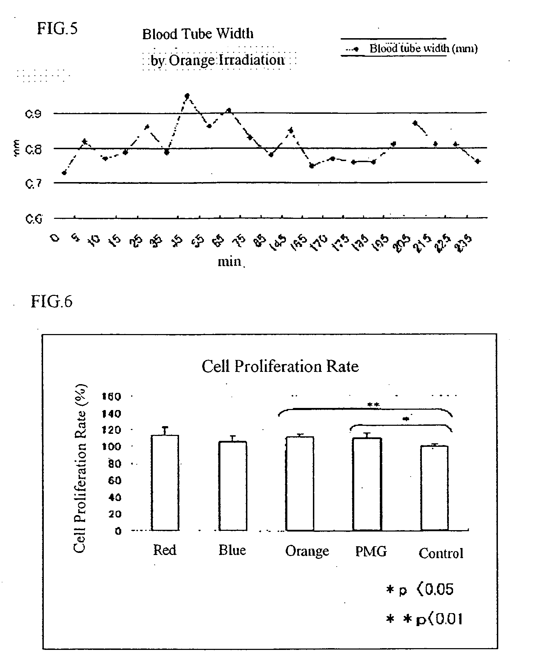

By irradiating the skin with radiation of chemical luminescence, the expansion of blood tube width as well as the improvement of blood flow was confirmed.
The chemical luminescence body of orange fluorescent light was used in this example.
The observation test to watch the blood tube width was performed for 235 hours in total, by exposure to orange fluorescent light for 50 minutes from 15 minutes to 65 minutes, and again for 20 minutes from 185 minutes to 205 minutes, having the interval of 100minutes, and the result was obtained as shown in Fig. 5 concerning the change of the blood tube width. It was found that the blood tube width started to expand 15 minutes after the exposure, remained in the expanded state from 45 minutes to 65 minutes, namely from 30 minutes after the exposure to the end of exposure, and then it returned to the original blood tube width after the end of the exposure, restarted to expand from 185minutes when the second exposure was resumed, continued to expand for 40 minutes, and returned to the normal width after 30minutes from the stop of exposure (205 minutes).
The measurement of the blood tube width was performed as described below:

Measurement method
Measurement is made at 23 degrees C and at humidity of 30% in the constant temperature and humidity controlled room.

1. The tranquil state is kept for 10 to 15 minutes in the controlled room and the measurement was made at the fixed position for the middle finger of the left hand.
2. The measurement was made for several times, exposing the stable site with orange fluorescent light for 50 minutes at the interval of 10 minutes.
3. About one hour later when the tranquil state is recovered, exposure to the orange fluorescent light was repeated again.
4. Measurement is performed for 20 minutes at the interval of 10 minutes.

The skin temperature change was measured by using the chemical luminescent body of red fluorescent light and by irradiating it to the skin of the forearm at two positions of the internal side, namely at position A at the elbow and at position B at the wrist. And it was found that the skin temperature rose and that the promotion of the transdermal absorption was promoted by using the external use skin agent at the same time. The result is shown in Table 3.
The average of five time monitoring shows that there is a temperature rise of 0.4 degrees C at position B and 0.7 degrees at position of the wrist by exposure to red fluorescent light.

[Table 3]

| Measurement of skin temperature by exposure to red fluorescence | | | |
|---|---|---|---|
| | Monitor | A Exposure position Observed temperature Degrees C | B Exposure position Observed temperature Degrees C |
| Before exposure | 1 | 31.9 | 31.7 |
| | 2 | 32.1 | 31.7 |
| | 3 | 32.1 | 31.7 |
| | 4 | 32.0 | 31.6 |
| | 5 | 32.1 | 31.8 |
| | Average | 32.0 | 31.7 |
| After exposure of Red fluorescent light For 5 minutes | 1 | 32.4 | 32.5 |
| | 2 | 32.4 | 32.5 |
| | 3 | 32.6 | 32.4 |
| | 4 | 32.3 | 32.3 |
| | 5 | 32.3 | 32.4 |
| | Average | 32.4 | 32.4 |

As the result, it was found that the exposure of the skin to irradiation of chemical luminescence had the effect to expand the blood tube width and to increase the skin temperature at the exposure site and at its vicinity.

<Cell proliferation>

By exposing the skin to three varieties of chemical luminescence, namely red fluorescent light, blue fluorescent light and orange fluorescent light, the observation was made to see how the cells proliferates. The result is shown in Fig. 6. The same rate of proliferation as PMG(employed as a measure for cell proliferation test) was observed by orange fluorescent light and red fluorescent light.
Incidentally, PMG stands for "phosphoric acid- L -magnesium ascorbate".
In comparison with the control, the cell proliferation was significantly promoted in the PMG addition system and in the orange fluorescent light system.

The test was performed in the procedure described below:

1. Cell: Normal human skin fibroblast (NHF)
2. Chemical fluorescent body: CYALUME Light shape blue, orange and red Seller: OmniGlow Japan Co., Ltd.
3. Assay:

(1) Seeded NHF to 24well plate with $2.0 \times 10^4$ cells/well, DMEM · 10%S(+)
(2) After confluent state is observed by culturing 5 days, it was transferred to non-color and non-blood-serum DMEM.
(3) Exposed to fluorescence (4 hrs.). 100mM PMG additive system was used as the positive control.
(4) Observed the appearance of the cell (x40)
(5) The quantitative measurement of cell proliferation was made by using MTT assay and by means of colorimetric test with wavelengths of 540nm for measurement and 655nm for control. The cell proliferation rate was calculated by using the formula described below, setting the untreated control as 100%.

4. Calculation formula

$$\text{Cell proliferation rate} = ([\text{Sample addition OD}(540nm)] - [\text{Sample addition OD}(655nm)]) /$$

$$([\text{Control OD}(540nm)] - [\text{Control OD}(655nm)]) \times 100$$

EXAMPLE 2

<u>Melanin synthesis by fluorescent light irradiation</u>

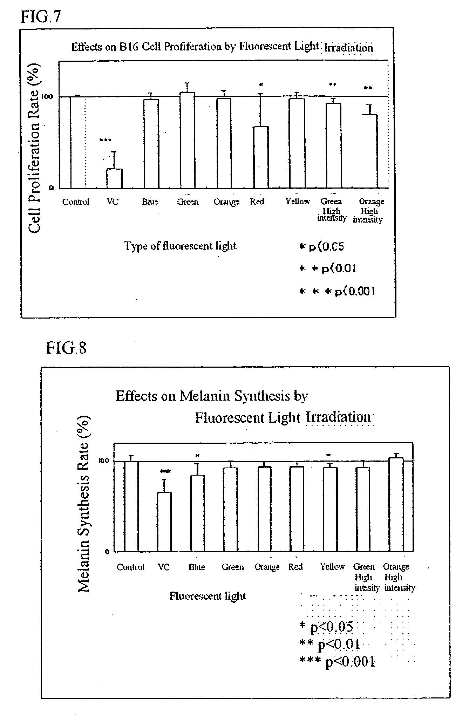

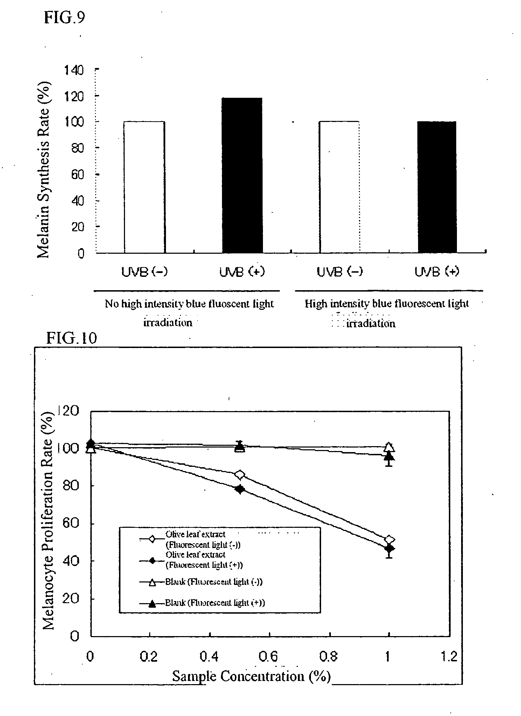

It was confirmed that exposure of the cell to fluorescent light irradiation had the effect to suppress the proliferation of the melanoma cell and the melanin synthesis. The influence upon the melanoma cell proliferation is shown in Table 4 and Fig. 7, and the influence upon the melanin synthesis of the cell is shown is shown in Table 5 and Fig. 8.
(Test method)

Cell: mouse melanoma(B16cell)
Chemical luminescentbody: Normal type

Blue fluorescent light, orange fluorescent light, red fluorescent light, green fluorescent light, and yellow fluorescent light
High intensity type
Orange fluorescent light, red fluorescent light, and green fluorescent light (Produced by OmniGlow Japan Co., Ltd.)

Assay:

1. Seeded B16 cell to 24well plate with 3.6 x 10$^{-3}$ cells/well, MEM · 10%S(+) · glucosamine culture medium. n=8
2. After culturing 5 days, it was transferred to MEM · 10%S(+) · theophyline culture medium.
3. Exposed to fluorescent light by placing the chemical luminescent body in contact with the plate for 4 hr. in the case of normal type and for 30 min. in the case of high intensity type. The control was under no exposure to fluorescent light and the positive control was in the ImMVC(Ascorbic acid) addition system.
4. The cell was recovered three days after the start of exposure, dissolved into 0.1 % DS normal saline solution, and then the optical density was measured at 260nm (index of the amount of cell) and at 475nm (index of the amount of melanin), and then the cell proliferation rate and the melanin synthesis rate per unit cell was calculated. The B16 cell proliferation rate is shown in Table 4 and Fig. 7, and the melanin synthesis rate per unit cell is shown in Table 5 and Fig. 8.

Calculation formula

$$\text{B16 cell proliferation rate(\%)} = \text{Sample Abs}(260nm) / \text{Control Abs}(260nm) \times 100$$

$$\text{Melanin synthesis rate per unit cell(\%)} = [\text{Sample Abs}(475nm) / \text{Sample Abs}(260nm)]$$

$$/ [\text{Control Abs}(475nm) / \text{Control Abs}(260nm)] \times 100$$

[Table 4]

| Treatment | Control | VC 1mM | Blue Normal | Green Normal | Orange Normal | Red Normal | Yellow Normal | Green High intensity | Orange High intensity |
|---|---|---|---|---|---|---|---|---|---|
| B16 cell proliferation rate(%) | 100 | 21.42 | 97.40 | 105.78 | 98.30 | 67.21 | 97.25 | 93.28 | 80.86 |
| S.D. | 2.78 | 18.87 | 7.42 | 10.37 | 8.99 | 36.38 | 7.17 | 4.75 | 11.10 |

(continued)

| Treatment | Control | VC 1mM | Blue Normal | Green Normal | Orange Normal | Red Normal | Yellow Normal | Green High intensity | Orange High intensity |
|---|---|---|---|---|---|---|---|---|---|
| Significant difference | - | $p<0.001$ | none | none | none | $p<0.05$ | none | $p<0.01$ | $p<0.01$ |

[Table 5]

| Treatment | Control | VC 1mM | Blue Normal | Green Normal | Orange Normal | Red Normal | Yellow Normal | Green High intensity | Orange High intensity |
|---|---|---|---|---|---|---|---|---|---|
| Melanin synthesis rate (%) | 100 | 65.28 | 85.12 | 92.77 | 93.88 | 94.08 | 92.62 | 92.48 | 103.86 |
| S.D. | 6.77 | 14.91 | 12.53 | 7.71 | 6.10 | 5.91 | 4.72 | 8.37 | 4.66 |
| Significant difference | - | $p<0.001$ | $p<0.05$ | none | none | none | $p<0.5$ | none | none |

Table 4 and Fig. 7 shows that the melanoma cell proliferation was suppressed significantly by exposure to normal type red fluorescent light, high intensity type green fluorescent light, and high intensity type orange fluorescent light. It is apparent that irradiating the skin with red fluorescent light, high intensity type green fluorescent light and orange fluorescent light has the effect to suppress the melanoma cell proliferation and melanocyte proliferation.
It is anticipated from this result that the present invention has the whitening effect.

Table 5 and Fig. 8 shows that the melanin synthesis per unit cell was suppressed significantly by exposure to blue fluorescent light, and yellow fluorescent light.

Unlike UV light, fluorescent light irradiation does not promotes the melanin synthesis, and it was found that blue and yellow fluorescent light has the significant effect to suppress the melanin synthesis. In particular, irradiation with blue fluorescent light has shown the melanin synthesis inhibiting effect at 20%.
The effect to suppress the melanocyte proliferation was recognized by normal type red fluorescent light, high intensity type green fluorescent light, and high intensity type orange fluorescent light. It is considered that the total melanin amount has decreased due to the decrease in the number of cells.
As fluorescent light irradiation did not restrain the fibroblast in terms of inhibiting the proliferation, it is understood to have selective action upon the activated melanocyte. Therefore, the said irradiation is considered useful for whitening.

EXAMPLE 3

<Melanin synthesis test >

(Test procedure)

The three dimensional skin model(melanoderm(MEL-300A))(produced by Mat Tek Co., Ltd.) is exposed to UVB irradiation of 1 J/m$^2$ at 1 W/m$^2$ and/or high intensity blue fluorescent light (produced by OmniGlow Japan Co.,Ltd.) for 1 hour after exposure to UV light for 0, 48, and 96 hours, and the cultured for 7 days.
The three dimensional skin model is boiled and dissolved in 1N sodium hydroxide, optical density is measured at 350nm to obtain the index for the melanin amount, and the melanin synthesis rate is calculated by the formula below. The result is shown in Fig. 9.
The solution containing the dissolved skin tissue not exposed to UVB nor to high intensity blue fluorescent light is prepared as the control.

Formula: [Melanin synthesis rate] = [Optical density at 350nm for soln. of treated tissue] / [Optical density at 350nm for soln. of control tissue] x 100

It was confirmed by using the three dimensional skin model that UVB irradiation promotes the melanin synthesis but irradiation of high intensity blue fluorescent light suppresses the promotion of melanin synthesis by UVB. Therefore, by irradiating the skin with the UV light such as the sunshine with fluorescent light like blue fluorescent light, melanin synthesis is suppressed and the generation of blackening, stains and freckles can be suppressed. Consequently, the fluorescent light irradiation is regarded useful for whitening as well as for anti-aging of the skin.

EXAMPLE 4

< Proliferation test of human epidermal melanocyte >

(Test procedure)

Human epidermal melanocyte is seeded to 24 well plate at 20000 cells / $cm^2$ by using the HMGS added base medium 154S for human epidermal melanin cells (made by Kurashiki Boseki Co., Ltd.).
After allowing incubation for 24 hours, the specimen is transferred to the medium added with olive leaf extract (refer to example below) or 50% aqueous solution of butyl alcohol as blank, exposed to high intensity blue fluorescent light (produced by OmniGlow Japan Co.Ltd.) and cultured for 72 hours.
After washing with PBS(-), the cell is dissolved into PBS(-) containing 1W/W% dodecil sodium sulfate, and then the optical density of the solution is measured at 260nm and the cell proliferation rate is calculated by the formula below.
The cell solution added with blank solution and not exposed to high intensity blue fluorescent light is prepared as the control.
The result is shown in Fig. 10 in the form of the relationship between the concentration of added olive extract and the melanocyte proliferation rate.
Olive Leaf Extract
(Example for preparation of olive leaf extract)
Dried olive leaf in the amount of 1 kg is immersed in 90W/W% ethanol for 1 week, concentrated and freeze- dried, and the dissolved into 50W/W% aqueous solution of butylene glycol in the concentration of 1 W/W%.

Formula: [Cell proliferation rate] = [Optical density (260nm) for soln. of specimen] / [Optical density at 260nm for soln. of control] x 100

There was no change in the proliferation of human epidermal melanocyte by 1 hour exposure to high intensity blue fluorescent light, but when olive leave extract was added to the specimen, there was a significant drop in the proliferation rate (upon addition of 0.5% olive extract addition) by irradiation with high intensity blue fluorescent light. The function of olive leaf extract to inhibit the proliferation rate human epidermal melanocyte was enhanced synergistically by using high intensity blue fluorescent light at the same time. Therefore, the exposure of the skin to blue fluorescent light has the effect to suppress the proliferation of human epidermal melanocyte. This means it is effective for whitening.

EXAMPLE 5

<Test of promotion about fibroblast proliferation and collagen synthesis>

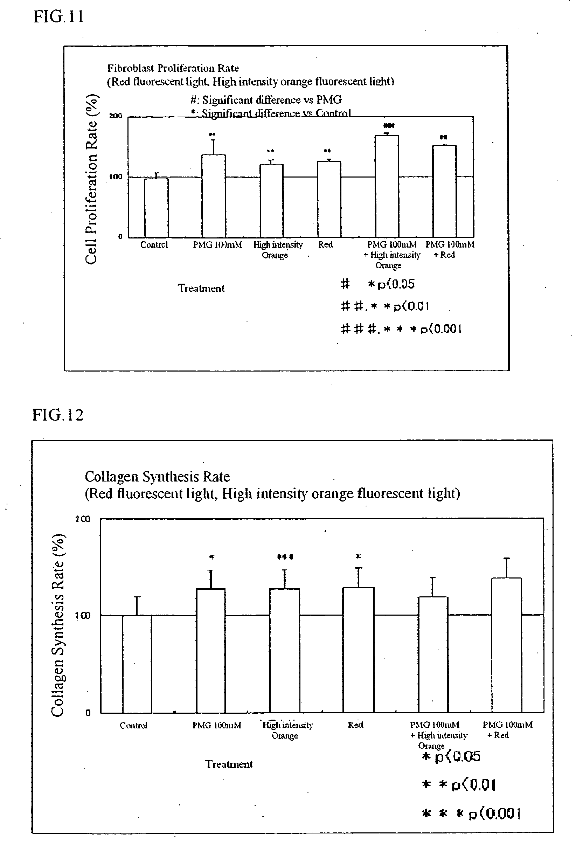

By exposing fibroblast to normal type red fluorescent light and high intensity orange fluorescent light, a test was made to see if it promotes the fibroblast proliferation and collagen synthesis of fibroblast. The test result is shown in Table 6 and Fig. I 1 for fibroblast and in Table 7 and Fig. 12 for collagen synthesis.
(Test procedure)

Cell: Fibroblast(NHF)

Chemical luminescence body (produced by OmniGlow Japan Co., Ltd.)
Normal type red (red fluorescent light)

High intensity orange (orange fluorescent light)

Collagen measurement kit: The amount of propeptide at the end of I type collagen C (PIP) is measured by Takara PIP kit.

Assay:

(1)NHF is seeded at $2.0 \times 10^4$ to 24 well plate with DMEM · 10%S(+).
(2) After confluent state is observed by culturing 5 days, it was transferred to non-color and non-blood-serum DMEM.
(3) Fluorescent light is allowed to irradiate (30min.) by placing the chemical luminescent body in direct contact with 24 well plate. 100mM PMG(phosphoric acid-L-magnesium ascorbate) addition system was prepared as the positive control.
(4) After 24 hours from the start of irradiation, the upper layer of the medium is recovered.
The amount of propeptide at the end of I type collagen C (PIP) is measured by Tatara PIP kit and then the colorimetric determination of cell proliferation is made by using MTT assay at observation wavelength of 540nm and at reference wavelength of 655nm. The cell proliferation rate is calculated by the formula below by setting the control of non-treated group as 100%.

$$\text{Cell proliferation rate} = ([\text{Sample addition OD(540nm)}] - [\text{Sample addition OD(655nm)}]) / ([\text{Control OD(540nm)}] - [\text{Control OD(655nm)}]) \times 100$$

The collagen synthesis rate is calculated by formula below by using the result of PIP amount and MTT Assay.

$$\text{Collagen synthesis rate} = \{\text{PIP amount of sample} / ([\text{Sample addition OD(540nm)}] - [\text{Sample addition OD(655nm)}])\} / \{\text{PIP amount of control} / ([\text{Control OD(540nm)}] - [\text{Control OD(655nm)}])\} \times 100$$

[Table 6]

| Treatment | Control | PMG 100μM 100mM | High intensity orange fluorescent light | Red fluorescent light | PMG 100Mm+ High intensity orange fluorescent light | PMG 100mM+ Red fluorescent light |
|---|---|---|---|---|---|---|
| Fibroblast proliferation rate(%) | 98.3 | 138.2 | 122.2 | 126.7 | 168.6 | 153.0 |
| S.D. | 8.6 | 23.0 | 6.4 | 3.4 | 4.0 | 2.3 |
| Significant difference VS control | $p<0.01$ | $p<0.01$ | $p<0.01$ | $p<0.01$ | $p<0.001$ | $p<0.01$ |
| Significant difference VS PMG | - | - | - | - | $p<0.001$ | $p<0.01$ |

From the test result of fibroblast proliferation shown in Table 6 and Fig. 11, it is seen that the fibroblast cell proliferation is significantly promoted by both normal type red fluorescent light and high intensity orange fluorescent light in the collagen synthesis promotion test system to measure cell proliferation rate after exposing 24 hours to normal type red fluorescent light and high intensity orange fluorescent light. Also in the case of combined treatment with PMG, the significant proliferation of the fibroblast was observed by exposure to normal type red fluorescent light and high intensity orange fluorescent light.

[Table 7]

| Treatment | Control | PMG 100mM | High intensity orange fluorescent light | Red fluorescent light | PMG 100Mm+ High intensity orange fluorescent light | PMG 100mM+ Red fluorescent light |
|---|---|---|---|---|---|---|
| Collagen synthesis rate (%) | 100.0 | 127.6 | 127.8 | 129.0 | 119.4 | 139.1 |
| S.D. | 6.2 | 35.8 | 12.9 | 15.5 | 3.4 | 12.2 |
| Significant difference — VS control | | $p < 0.05$ | $p < 0.001$ | $p < 0.05$ | $P < 0.001$ | $p < 0.01$ |
| Significant difference — VS PMG | | - | - | - | none | none |

From the measured data shown in Table 7 and Fig. 12, it is recognized that the collagen synthesis rate per unit cell is accelerated significantly to the same degree as PMG in the system of the exposure to normal type red fluorescent light and high intensity orange fluorescent light.

By exposing fibroblast to normal type red fluorescent light and high intensity orange fluorescent light, fibroblast proliferation and collagen synthesis are promoted. Further, by the combined use with PMG, fibroblast proliferation and collagen synthesis are still more accelerated. Therefore, irradiation with chemical luminescence like normal type red fluorescent light and high intensity orange fluorescent light has the effect to increase the collagen content in the skin and improves tension, wrinkles, elasticity and softness, and therefore it is expected to inhibit aging. Furthermore, the synergistic improvement effect is anticipated by the combined use with external use skin medicine containing PMG.

EXAMPLE 6

<fibroblast proliferation by exposure to fluorescent light>

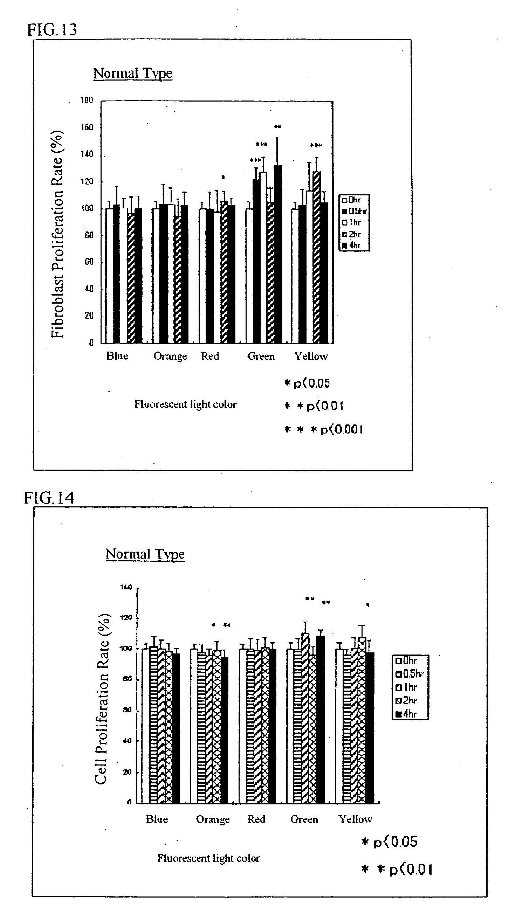

By exposing fibroblast to various types and varieties of fluorescent light, its influence upon the proliferation of fibroblast was examined. The result obtained by the test shown below is indicated in Fig. 13, 14 and 15. The summary of the evaluation result is shown in Table 8.

(Test procedure)

Cell: fibroblast (NHF)

Chemical luminescence body: (produced by OmniGlow Japan Co., Ltd.)
Normal type -blue fluorescent light, orange fluorescent light, red fluorescent light, yellow fluorescent light, and green fluorescent light.
High intensity orange fluorescent light, and high intensity green fluorescent light

Assay:

1. NHF is seeded at $2.5 \times 10^4$ cells/ml to 96 well plate with DMEM·10%S(+).

2. Non-color DMEM preparation 5%, the next day, and transferred to the medium containing 1%FBS.

3. Exposed to fluorescence by placing the chemical fluorescent body in contact with the plate.
Fluorescence irradiation (0.5, 1, 2, 4 hr., only 0.5hr. for high radiance type)
Culturing for 5 days at 5%CO and 37 degrees C.

4. The colorimetric determination is performed by using MTT assay at the observation wavelength of 540nm and the reference wavelength of 655nm.

The cell proliferation rate is calculated by the formula below by setting the control of non-treatment group as 100%.

$$\text{Cell proliferation rate} = ([\text{Sample addition OD(540nm)}] - [\text{Sample addition OD(655nm)}]) / ([\text{Control OD(540nm)}] - [\text{Control OD(655nm)}]) \times 100.$$

[Table 8]

| DMEM culture medium | | 1%FBS | | | | 5%FBS | | | |
|---|---|---|---|---|---|---|---|---|---|
| irradiation time(hr) | | 0.5 | 1 | 2 | 4 | 0.5 | 1 | 2 | 4 |
| Normal | Blue | | | | | | | | |
| | Orange | | | | | | (*) | | (**) |
| | Red | | | * | | | | | |
| | Green | *** | *** | | ** | | ** | | ** |
| | Yellow | | | *** | | | | * | |
| High intensity | Orange | * | | | | | | | |
| | Green | ** | | | | *** | | | |
| Significant difference (()indicates negative significance) | | | | | | | | | |

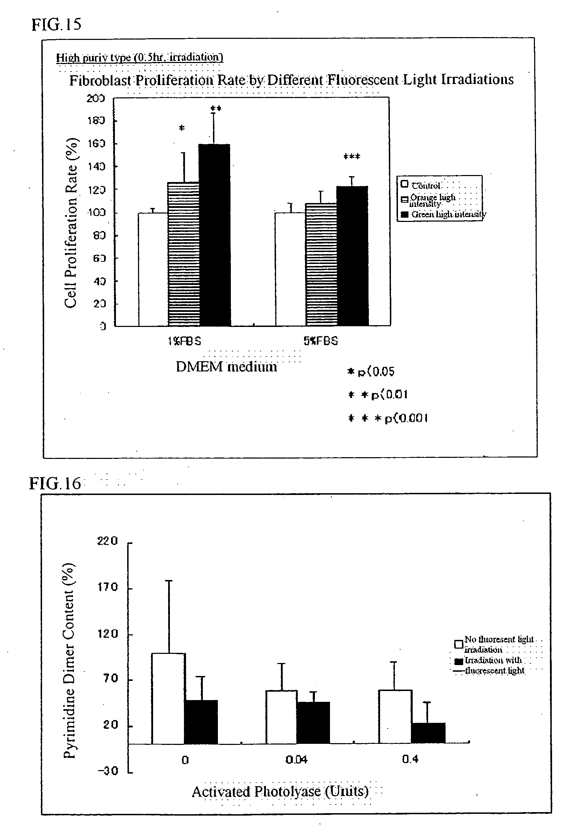

From the data in Fig. 13, Fig.14 and Table 8 showing the test result of normal type fluorescent light irradiation, the significant effect to promote the cell proliferation was recognized at 5% FBS medium by 1 hr., and 4 hr. irradiation with green fluorescent light and by 2 hr. irradiation with yellow fluorescent light.
In the case of 1 % FBS medium, the significant effect to promote the cell proliferation was recognized by 2 hr. irradiation with red fluorescent light, by 0.5hr., 1 hr., and 4 hr. irradiation with green fluorescent light and by 2 hr. irradiation with yellow fluorescent light.
In the test of yellow fluorescent light, the tendency of increase was observed in accordance to the irradiation time up to 2 hr.
From the data of Fig. 15 and Table 8 showing the test result of high intensity fluorescent light irradiation (0.5 hr. irradiation), the significant effect to promote the cell proliferation was recognized at 1% FBS · DMEM medium by orange fluorescent light and green fluorescent light. In particular, the proliferation was about 1.5 times.
In the case of 5 % FBS medium, the significant effect to promote the cell proliferation was recognized only by irradiation with green fluorescent light,
[0058] In this test, the effect to promote the cell proliferation was higher when the FBS concentration was lower.
The cell activation effect was observed by normal type red fluorescent light, green fluorescent light and yellow fluorescent light, and in particular, the said effect was seen by green fluorescent light even in the case of high FBS concentration.
The cell activation effect was observed also by high intensity orange fluorescent light and green fluorescent light, and also in the case of high FBS concentration, the activation effect was seen by green fluorescent light. The test result described above suggests that the fluorescent light color most likely to be active is blue.

EXAMPLE7

<Activity improvement test of photolyase>

When the cell is exposed to UVB irradiation, DNA is damaged and pyrimidine dimer is produced. The repeated exposure causes the progress of light-aging. It is known that Prokayote, having the capability of photosynthesis, possesses "photolyase" which can repair the dimerization of DNA base and that photolyase is activated by the visible light. (Fragrance Journal, June, 2002 P49-53) It is reported that this enzyme is active to the visible light, but nothing is mentioned about the effect by the fluorescence luminous body.
An investigation was made to see the action of high intensity blue fluorescent light irradiation upon the activity of photolyase, and also to see the effect by the combined use of the chemical luminescence body with the active elements of cosmetics.

(Test procedure)

Seeded human keratinocyte of $7.0 \times 10^5$ cells to 60mm dia. dish. After incubating overnight, it is transferred to Hanks(+) and exposed to UVB of $0.5 \text{J/m}^2 (1\text{W/m}^2)$. It is then transferred to Epilife added with Humedia-KG (made by Kurashiki Boseki Co., Ltd.), added with a sample (50% butylenes glycol for blank, 0.04Units, 0.4Units (pyrimidine dimer repairing activity) for photolyase) and exposed to high intensity blue fluorescent light (produced by OmniGlow Japan Co., Ltd.) for 1 hr. A control is prepared without adding photolyase and without exposing to high intensity blue fluorescent light.

After exposure to UVB for 6 hours, DNA is recovered from the cell in accordance with the normal method by using DNAzol reagent (made by Inbitrogen Co., Ltd.).
The amount of pyrimidine dimer observed in the measurement is shown in Fig. 16.

(Measurement method of pyrimidine dimer)

The amount of pyrimidine dimer is measured by the procedure described below by using DNA of this test.
Liquid of 1% protamine sulfate is seeded to 96 well plate and incubated at 37 degrees C for 2 hours. After washing the plate with sterile water and drying it, 2 $\mu$g of abstracted DNA is seeded to each well, and incubated at 37 degrees C for 20 hours. Then, after washing with PBS containing 0.05%Tween20 (PBS-T) and adding PBS containing 2% skim milk, it is incubated at 37 degrees C for 1 hour. Further, it is incubated at 37 degrees C for 1 hour by adding thymine dimer monoclonal antibody prepared by PBS containing 2% skim milk. After that, it is washed with PBS-T, seeded with HRP bonded goat antimouse IgB antibody, and incubated at 37 degrees C for 1 hour. Then, after washing with PBS (PBS-T), it is washed with PBS(-). 3,3',5,5' tetramethylbenzidine solution (1-StepTMTurbo TMB-ELISA(made by Pierce Co., Ltd.) is added, incubation is performed at room temperature, and then the reaction is stopped by 2N sulfuric acid. After measuring the optical density at 450nm, the content of pyrimidine dimer per 2 $\mu$g of DNA is calculated by the formula below. Anacystis nidulans of cyanobacteria genus is used as photolyase, and PHOTOSOMES® made by AGI Dermatics Co., Ltd. Containing 41 Units/mL of photolyase is added.

Formula: [Pyrimidine dimer content(%)]=[Sample addition Optical Density (450nm)] / ([Control Optical Density (450nm) ] x 100

The measured content of pyrimidine dimer is shown in Fig. 16.
In the photolyase non-addition system, the pyrimidine dimer content dropped to almost 1/2 by exposing to high intensity blue fluorescent light.
By adding either 0.04Unit or 0.4 Unit of photolyase, the pyrimidine dimer content dropped to almost 1/2 against the control, but there was no difference in the effect to pyrimidine dimer content decrease whether the photolyase addition was 0.04 Unit or 0.4 Unit - increase to ten times. But, by the combined use of high intensity blue fluorescent light with photolyase, the content of pyrimidine dimer decreases and, in particular, it was possible to reduce the pyrimidine dimer to about 1/4 by the combined use of 0.4 Unit and high intensity blue fluorescent light.
The content of pyrimidine dimer was reduced significantly in comparison with the control by irradiation with high intensity blue fluorescent light, the combined use of 0.4 Unit of phtolyase and high intensity blue fluorescent light, and the combined use of 0.4 Unit of phtolyase and high intensity blue fluorescent light. Therefore, by the combined use of phtolyase and high intensity blue fluorescent light, light-aging can be suppressed synergistically.
In particular, light-aging can be restrained by using either chemical luminescence irradiation or by the action of photolyase. The DNA damage of the skin cell by UV light and the light-aging can be suppressed by irradiation with high intensity blue fluorescent light or by its use in combination with photolyase.

EXAMPLE 8

<Blood flow increase test by near-infrared rays of chemical fluorescence >

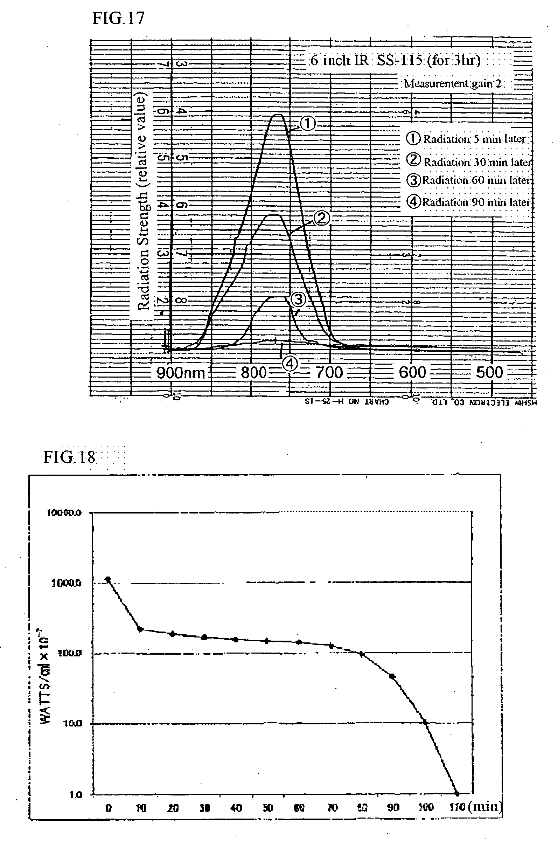

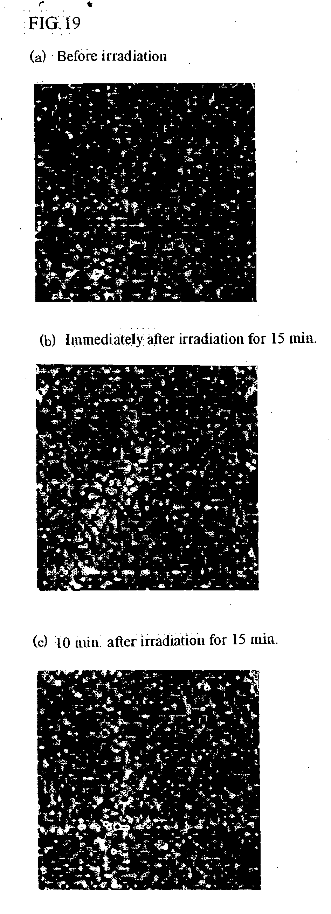

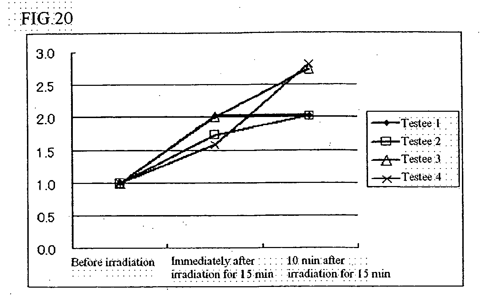

The change of blood flow was observed by exposing the skin to near-infrared rays obtained by chemical luminescence.
An example of the near-infrared rays observed by chemical luminescence is shown in Fig. 17 and 18.
The test conditions, an example of the image and the blood flow change of the testee are shown in Table 9, Fig. 19 and Fig. 20, respectively.
[Table 9]

| Test of human cell exposure to near-infrared chemical luminescence body | |
|---|---|
| Measurement conditions | All weather room was used.<br>Humidity 35%<br>Room Temp. 24 degrees C +3<br>Outside light was blocked as much as possible.<br>(No illumination was used during measurement.) |
| IR chemical luminescence body Blank | Custom-made product by OmniGlow Japan Co., Ltd.<br>CYALUME 6 inches stick type<br>Non-activated OmniGlow Japan Co., Ltd. CYALUME 6 inches stick type |
| Method of exposure | The stick was swung up and down five times after it is lit. And then the skin was exposed to irradiation in direct contact with it. |
| Number of testees | Five persons.<br>They entered the all weather room ten minutes before the measurement and stayed there to calm. |
| Measurement instrument | PIM II laser doppler of Perimed Co., Ltd. and blood flow meter |
| Measurement positions | Left arm, 5 cm away from measurement head<br>Measurement area 3cm x 3 cm |
| IR chemical luminescence body | Near-infrared rays |
| No. of measurement | 3 times<br>1. Before the irradiation<br>2. Upon irradiation for 15 min. by near-infrared chemical luminescence body<br>3. 10 min. after irradiation is completed |
| Image treatment | A tester adjusts the initial value.<br>The image change is observed by fixing the initial value in the 2nd and 3rd measurement |
| Image judgment | The more the blood flow, the brighter the image display. |
| Image analysis | The average of the brightness was calculated.<br>By setting the brightness before near-infrared irradiation to 1, the brightness after irradiation was calculated and compared. |

Fig. 19 (a) (b) and (c) show the image of a testee taken before measurement, immediately after 15 min. exposure, and upon 10 min. after 15 min. exposure respectively. Growing of the bright spots is recognized in the image and it is seen that the blood flow remains increased even when 10 min. has passed after the exposure.
From the graph in Fig. 20 showing the change of the blood flow of testees by the numerical values converted from the image, it is observed that the blood flow after the exposure is increased to 1.5 to 3 times than the one before the exposure. The blood flow upon 10 min. after the stop of exposure remained increased for all testees without exception, and thus the exposure for a short time proved to have the effect to act for a long time.

EXAMPLE 9

<Trial use test>

The green fluorescent light pack was used which is made of photo-transmitting film shaped in an oval with the short side of 50mm and the long side of 70mm and with thickness of 8mm in the central area. As the reference, a non-lighting fake pack of the same shape was used.
To each of five testees, a non-lighting fake pack (blank) was applied on the right cheek and a fluorescent light pack of blue irradiation on the left. The test was done for five days, by putting the pack to both right and left cheeks once a day for 30 minutes at night. The result is shown in Table 10.

The result indicates that all testees felt the improved skin tension already on the 1st day, with 3 testees feeling better on the 3rd day and 4 testees on the 5th day. There was no one who did not feel the better feeling. Therefore, it has been confirmed that the fluorescent light irradiation by chemical luminescence has the effect to improve the skin tension. There was no one who felt anything not normal by exposure to the green fluorescent light.

[Table 10]

|  | Felt | Felt a little | Not felt |
|---|---|---|---|
| 1st day |  | 5 |  |
| 3rd day | 3 | 2 |  |
| 5th day | 4 | 1 |  |

**Claims**

1. A method for beauty treatment or makeup by exposing a skin to the light obtained by chemical luminescence.

2. The method for beauty treatment or makeup according to claim 1, **characterized in that** the light is selected from the wavelength range of 300 to 1200nm.

3. The method for beauty treatment or makeup according to claim 1 or 2, **characterized in that** the skin is a part of the face or the body.

4. The method for beauty treatment or makeup according to any one of claims 1 to 3 , **characterized in that** the chemical luminescence is generated by mixing two liquid components.

5. The method for beauty treatment or makeup according to any one of claims 1 to 4 , **characterized in that** the light is irradiated onto an applied area after an external use skin medicine is applied or while a pack agent is being applied.

6. The method for beauty treatment or makeup according to claim 5, **characterized in that** the external use skin medicine or the pack agent comprises a composition for beauty treatment or for makeup.

7. A luminous body for irradiating a skin for beauty treatment or makeup, **characterized in that** a chemical luminescent reagent is contained.

8. The luminous body for irradiating the skin for beauty treatment or makeup according to claim 7, **characterized in that** the chemical luminescent reagent constituted by two liquid components that emits light upon mixing, is stored separately in one container, and mixed to expose the skin upon use.

9. The luminous body for irradiating the skin for beauty treatment or makeup according to claim 7 or 8, **characterized in that** the light for irradiating the skin is selected from the wavelength range of 300 to 1200nm.

10. A tool for beauty treatment or makeup, **characterized in that** an external use skin medicine or oral medicine is used in combination with the luminous body for irradiating the skin for beauty treatment or makeup according to any one of claims 7 to 9.

11. A method for whitening makeup or whitening beauty treatment by exposing a skin to the light of chemical luminescence.

12. The method for whitening makeup or whitening beauty treatment according to claim 11, **characterized in that** the growth of melanin producing cells and / or the synthesis of melanin is inhibited by exposing the skin to the light of chemical luminescence.

13. The method for whitening makeup or whitening beauty treatment according to claim 11 or 12, **characterized in that** a luminous color of the chemical luminescence comprises one or a plurality of a combination selected from blue fluorescent light, yellow fluorescent light, green fluorescent light, orange fluorescent light and red fluorescent light.

14. A whitening luminous body containing a luminescent reagent that emits the light of chemical luminescence to a skin.

15. The luminous body according to claim 14, **characterized in that** the luminescent reagent inhibits the growth of melanin producing cells and / or the synthesis of melanin by emitting the light of chemical luminescence to the skin.

16. The luminous body according to claim 14 or 15, **characterized in that** the luminescent reagent emits fluorescent light comprising one or a plurality of a combination selected from blue fluorescent light, yellow fluorescent light, green fluorescent light, orange fluorescent light and red fluorescent light.

17. The method for whitening makeup or whitening beauty treatment according to any one of claims 11 to 13, **characterized in that** the blue fluorescent light is irradiated onto the skin as chemical luminescence while olive leaf extract is being applied on the skin, being applied as a pack agent or after taking it orally.

18. A tool for whitening makeup or whitening beauty treatment, **characterized by** comprising a combination of a whitening luminous body containing a chemical luminescent reagent of blue fluorescent light and an external use skin medicine, a pack agent and / or an oral medicine containing olive leaf extract.

19. A beauty treatment method or a makeup method, **characterized in that** the growth of fibroblast and / or the synthesis of collagen is promoted by exposing a skin to the light of chemical luminescence comprising one or a plurality of orange fluorescent light, red fluorescent light, green fluorescent light and yellow fluorescent light.

20. The method for beauty treatment or makeup according to claim 19, **characterized in that** the growth of fibroblast and / or the synthesis of collagen is promoted by exposing the skin to the light of chemical luminescence comprising one or a plurality of orange fluorescent light, red fluorescent light, green fluorescent light, and yellow fluorescent light while ascorbic acid, its salt and / or a derivative of ascorbic acid are being applied on the skin, being applied as a pack agent or after taking it orally.

21. A luminous body, **characterized in that** a luminescent reagent that emits fluorescent light comprising one or a plurality of orange fluorescent light, red fluorescent light, green fluorescent light and yellow fluorescent light as the light of chemical luminescence to promote the growth of fibroblast and / or the synthesis of collagen by exposing a skin is contained.

22. A tool for beauty treatment or makeup, **characterized in that** the tool comprises a combination of the luminous body according to claim 21 with an external use skin medicine, a pack agent and / or an oral medicine containing ascorbic acid, or its salt and / or a derivative of ascorbic acid.

23. A light anti-aging method by exposing a skin to blue fluorescent light.

24. The light anti-aging method according to claim 23 , **characterized in that** generating pyridine dimer is inhibited by exposing the skin to the blue fluorescent light of chemical luminescence.

25. The light anti-aging method according to claim 23 or 24, **characterized in that** the skin is exposed to the blue fluorescent light while photolyase is being applied on the skin, being applied as a pack agent or after taking it orally.

26. A luminous body for light anti-aging, **characterized by** containing a chemical luminescent reagent to irradiate a skin with blue fluorescent light.

27. A light anti-aging tool for makeup or beauty treatment, **characterized by** comprising a combination of a chemical luminescent reagent that radiates blue fluorescent light to a skin and an external use skin medicine, a pack agent and / or an oral medicine containing photolyase.

28. A method to increase blood flow by exposing a skin to fluorescent light of chemical luminescence and / or near-infrared rays of chemical luminescence.

29. The method to increase the blood flow according to claim 28, **characterized in that** the fluorescent light of the chemical luminescence is orange fluorescent light.

**30.** A luminous body to increase blood flow, which contains a chemical luminescent reagent that emits fluorescent light and / or near-infrared rays.

**31.** The luminous body to increase the blood flow according to claim 30, **characterized in that** the fluorescent light is orange fluorescent light.

**32.** A tool for makeup or beauty treatment, **characterized by** comprising a combination of the luminous body to increase the blood flow according to claim 30 or 31 with an external use skin medicine.

**33.** The tool for makeup or beauty treatment according to any one of claims 10, 18, 22, 27, and 32, **characterized in that** the external use skin medicine is skincare cosmetics, face pack, poultice, patches, or transdermal absorbents.

**34.** A skin masking material, **characterized in that** any one of the luminous body or the tool for beauty treatment or makeup according to any one of claims 7, 8, 9, 10, 14, 15, 16, 18, 21, 22, 26, 27, 30, 31, and 32 is used for face masking or for body masking.

FIG. 1(a)

FIG. 1(b)

FIG.1(c)

FIG.1(d)

FIG.2

FIG.3

FIG.4

FIG.5

Blood Tube Width
by Orange Irradiation

--- Blood tube width (mm)

FIG.6

Cell Proliferation Rate

* p < 0.05

* * p < 0.01

FIG.7

FIG.8

FIG.9

Melanin Synthesis Rate (%)

UVB (−)  UVB (+)  UVB (−)  UVB (+)

No high intensity blue fluoscent light irradiation

High intensity blue fluorescent light irradiation

FIG.10

Olive leaf extract
(Fluorescent light (-))
Olive leaf extract
(Fluorescent light (+))
Blank (Fluorescent light (-))
Blank (Fluorescent light (+))

Melanocyte Proliferation Rate (%)

Sample Concentration (%)

FIG.11

Fibroblast Proliferation Rate
(Red fluorescent light, High intensity orange fluorescent light)

#: Significant difference vs PMG
*: Significant difference vs Control

# *p<0.05
##.**p<0.01
###.***p<0.001

FIG.12

Collagen Synthesis Rate
(Red fluorescent light, High intensity orange fluorescent light)

*p<0.05
**p<0.01
***p<0.001

**FIG.13**

**FIG.14**

FIG.15

High puriy type (0.5hr, irradiation)

Fibroblast Proliferation Rate by Different Fluorescent Light Irradiations

* p<0.05

* * p<0.01

* * * p<0.001

FIG.16

FIG.17

FIG.18

FIG. 19

(a) Before irradiation

(b) Immediately after irradiation for 15 min.

(c) 10 min. after irradiation for 15 min.

FIG.20

## INTERNATIONAL SEARCH REPORT

| | International application No. |
|---|---|
| | PCT/JP2005/002079 |

A. CLASSIFICATION OF SUBJECT MATTER
    Int.Cl⁷  A61N5/06, A61K7/00, 7/48

According to International Patent Classification (IPC) or to both national classification and IPC

B.  FIELDS SEARCHED

Minimum documentation searched (classification system followed by classification symbols)
    Int.Cl⁷  A61N5/06, A61K7/00, 7/48

Documentation searched other than minimum documentation to the extent that such documents are included in the fields searched
    Jitsuyo Shinan Koho          1922-1996    Jitsuyo Shinan Toroku Koho    1996-2005
    Kokai Jitsuyo Shinan Koho    1971-2005    Toroku Jitsuyo Shinan Koho    1994-2005

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br>Y | JP 2002-85575 A  (Otsuka Pharmaceutical Factory, Inc.),<br>26 March, 2002 (26.03.02),<br>All pages; Fig. 1<br>(Family: none) | 23<br>7-10,14-16,<br>18,22,24,26,<br>33 |
| Y | JP 2002-138278 A  (Nippon Omuniguro Kabushiki Kaisha),<br>14 May, 2002 (14.05.02),<br>Par. Nos. [0002], [0010], [0011]<br>(Family: none) | 7-10,14-16,<br>18,21,22,24,<br>26,30-33 |
| Y | JP 2003-313106 A  (Kabushiki Kaisha Santeberu),<br>06 November, 2003 (06.11.03),<br>Claim 1<br>(Family: none) | 18 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    20 April, 2005 (20.04.05) | Date of mailing of the international search report<br>    17 May, 2005 (17.05.05) |
|---|---|
| Name and mailing address of the ISA/<br>    Japanese Patent Office | Authorized officer |
| Facsimile No. | Telephone No. |

Form PCT/ISA/210 (second sheet) (January 2004)

## INTERNATIONAL SEARCH REPORT

International application No.

PCT/JP2005/002079

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-501169 A (ICN PHOTONICS LTD.), 14 January, 2003 (14.01.03), All pages; Figs. 1 to 6 & WO 2000/074782 A1 & EP 1183072 A | 21 |
| Y | JP 2003-137807 A (Miyagi Kagaku Kogyo Kabushiki Kaisha), 14 May, 2003 (14.05.03), Par. Nos. [0001], [0035]; Figs. 1 to 6 (Family: none) | 22 |
| Y | JP 2001-212250 A (Japan Science and Technology Corp.), 07 August, 2001 (07.08.01), Claim 1; Par. No. [0007]; Figs. 1 to 7 & WO 2001/056657 A1 | 30-32 |
| Y | JP 2003-12487 A (YAMAN Ltd.), 15 January, 2003 (15.01.03), All pages; Fig. 1 (Family: none) | 33 |
| A | JP 2003-88527 A (Aloka Co., Ltd.), 25 March, 2003 (25.03.03), Par. No. [0050]; Figs. 1 to 7 (Family: none) | 10,18,22,32, 33 |
| A | JP 2003-261787 A (Nihon University), 19 September, 2003 (19.09.03), Par. No. [0002] (Family: none) | 10,18,22,32 |
| A | JP 10-309323 A (Onkyo Corp.), 24 November, 1998 (24.11.98), All pages; Figs. 1 to 4 (Family: none) | 10,18,22,32 |

Form PCT/ISA/210 (continuation of second sheet) (January 2004)

## INTERNATIONAL SEARCH REPORT

| | International application No. |
|---|---|
| | PCT/JP2005/002079 |

---

**Box No. II**      Observations where certain claims were found unsearchable (Continuation of item 2 of first sheet)

---

This international search report has not been established in respect of certain claims under Article 17(2)(a) for the following reasons:

1. [×] Claims Nos.: 1-6, 11-13, 17, 19, 20, 25, 28, 29
because they relate to subject matter not required to be searched by this Authority, namely:
The description of claim 28 pertains to methods for treatment of a human body by therapy and thus relates to a subject matter which this International Searching Authority is not required to search under the provisions of PCT Article 17(2)(a)(i) and Rule 39.1(iv). (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

---

**Box No. III**      Observations where unity of invention is lacking (Continuation of item 3 of first sheet)

---

This International Searching Authority found multiple inventions in this international application, as follows:
While the special technical feature of claims 7-10 is a luminant characterized by "containing a chemiluminescent agent", the special technical feature of claims 23-25 is irradiation with "blue fluorescence". Whether or not the luminant contains a chemiluminescent agent is not involved in the constitution thereof.
Therefore, it appears that between these two inventions, one or more of the same or corresponding special technical features are lacking.

1. [ ] As all required additional search fees were timely paid by the applicant, this international search report covers all searchable claims.

2. [×] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**      [ ]   The additional search fees were accompanied by the applicant's protest.

                                     [ ]   No protest accompanied the payment of additional search fees.

---

Form PCT/ISA/210 (continuation of first sheet (2)) (January 2004)

**INTERNATIONAL SEARCH REPORT**

International application No.

PCT/JP2005/002079

Continuation of Box No.II-1 of continuation of first sheet(2)

Referring to claim 29, it describes irradiation with orange fluorescence. Thus, with respect to claims 1-6, 11-13, 17, 19 and 20 as well, it appears that similar methods are involved. Further, it appears that claim 25, as the expression "irradiating the skin with blue fluorescence after administration" appears and thus a method of administration is described, pertains to methods for treatment of a human body by therapy.

Form PCT/ISA/210 (extra sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004733 A
- WO 200312487 A
- WO H11342213 A
- JP 2003238823 A
- WO 2002138278 A
- WO H11045602 A
- WO 2003532253 A

**Non-patent literature cited in the description**

- *Fragrance Journal,* June 2002, 49-53